# EUROPEAN PATENT APPLICATION

(11) **EP 3 895 718 A1**
(43) Date of publication of application: **20.10.2021**
(21) Application number: 19896247.4
(22) Date of filing: 04.12.2019
(51) Int. Cl.: A61K 35/747, A61P 35/00, A23L 33/135, A23K 10/18, C12N 1/20, A23K 10/16, A23L 29/00, C12R 1/225

(54) **LACTOBACILLUS FERMENTUM WIKIM0102 HAVING ANTICANCER ACTIVITY AND COMPOSITION COMPRISING SAME AS EFFECTIVE COMPONENT**

(30) Priority: 10.12.2018 KR 20180158319; 25.06.2019 KR 20190075612
(71) Applicant: Korea Food Research Institute, Wanju-gun, Jeollabuk-do 55365 (KR)
(72) Inventor: CHOI, Hak Jong, Gwangju 62065 (KR); YUN, Mi Sun, Gwangju 62065 (KR)
(74) Representative: Pons
(86) International application number: PCT/KR2019/017020
(87) International publication number: WO 2020/122496

(57) **Abstract**

The present invention relates to a novel Lactobacillus fermentum WiKim0102 (Accession No. KCCM12356P) isolated from kimchi and a composition comprising the same as an active ingredient. Lactobacillus fermentum WiKim0102 according to the present invention shows excellent anti-cancer activity in conventional animal models and animal models in which a human cancer cell is transplanted, and therefore it can be usefully used as a composition in use of treatment, prevention or improvement of cancer of humans or animals.

## Description

### [TECHNICAL FIELD]

The present invention relates to novel Lactobacillus fermentum WiKim0102 (Accession No. KCCM12356P) isolated from kimchi and a composition comprising the same as an active ingredient.

### [BACKGROUND ART] nn

Cancer shows a high mortality rate worldwide, and is the most common cause of death after cardiovascular disease in Western societies. In particular, due to westernization of dietary habits, the intake of high-fat diets has become common, and because of a rapid increase in environmental pollutants and an increase in alcohol consumption, and the like, colon cancer, breast cancer, prostate cancer and the like continue to increase, and lung cancer is increasing due to an increase of smoking population and air pollution in addition to aging of the population. In this situation, creation of anti-cancer substances which can contribute to enhancement of human health, improvement of healthy quality of life and improvement of human health, by enabling early prevention and treatment of cancer is urgently required.

Meanwhile, lactic acid bacteria are widely distributed in mouth, intestine, vagina and feces of humans and animals and fermented foods such as kimchi, and are closely related to the health of humans and animals. Lactic acid bacteria show various health promotion effects such as intestinal regulation, inhibition of harmful bacteria, immunoregulation, lowering of cholesterol in blood, anti-cancer activity, and the like.

Currently, Korean Patent Publication No. 10-2015-0068061 discloses the anti-cancer activity of Lactobacillus plantarum PNU (KCCM11352P) or Lactobacillus mesenteroides PNU (KCCM11353P), and Korean Patent No. 10-1287120 discloses a pharmaceutical composition for treatment of cancer containing Lactobacillus plantarum DSR CK10 [Accession No.: KFCC-11433P] or Lactobacillus plantarum DSR M2[Accession No.: KFCC-11432P] as an active ingredient, but the anti-cancer activity and superiority of Lactobacillus fermentum have not been reported.

### [DISCLOSURE]

### [TECHNICAL PROBLEM]

An object of the present invention is to provide a novel kimchi lactic acid bacterium, Lactobacillus fermentum strain with excellent anti-cancer activity.

In addition, another object of the present invention is to provide a novel pharmaceutical composition for prevention or treatment of cancer comprising a novel kimchi lactic acid bacterium, Lactobacillus fermentum strain as an active ingredient.

Furthermore, other object of the present invention is to provide a food composition or food additive composition for prevention or improvement of cancer comprising a novel kimchi lactic acid bacterium, Lactobacillus fermentum strain as an active ingredient.

Moreover, other object of the present invention is to provide a feed composition or feed additive composition for prevention or improvement of cancer comprising a novel kimchi lactic acid bacterium, Lactobacillus fermentum strain as an active ingredient.

In addition, other object of the present invention is to provide a method for prevention or treatment of cancer, in which the composition is administered to a subject other than humans.

### [TECHNICAL SOLUTION]

Accordingly, the present inventors have tried to find a lactic acid bacteria strain that exhibits excellent effects as a probiotic and shows effects of prevention and treatment of cancer from kimchi, have isolated and identified a novel Lactobacillus sp. lactic acid bacteria strain having anti-cancer activity, Lactobacillus fermentum WiKim0102, thereby completing the present invention.

In order to achieve the objects, the present invention provides Lactobacillus fermentum WiKim0102 strain with excellent anti-cancer activity.

The Lactobacillus fermentum WiKim0102 is a novel strain of Lactobacillus fermentum derived from kimchi. Although Lactobacillus fermentum WiKim0102 of the present invention is isolated and identified from kimchi, but the means of acquiring is not limited thereto.

The Lactobacillus fermentum WiKim0102 of the present invention has a general intestinal regulation effect and an immune stimulating effect of lactic acid bacteria as a probiotic. It is a well-known fact that Lactobacillus sp. lactic acid bacteria have an intestinal regulation effect and an immune stimulating effect.

In addition, the present invention provides a pharmaceutical composition for prevention or treatment of cancer comprising Lactobacillus fermentum WiKim0102 (accession number KCCM12356P) or its culture as an active ingredient.

Furthermore, the present invention provides a food composition or food additive composition for prevention of improvement of cancer comprising Lactobacillus fermentum WiKim0102 (accession number KCCM12356P) or its culture as an active ingredient.

Moreover, the present invention provides a feed composition or feed additive composition for prevention or improvement of cancer of livestock comprising Lactobacillus fermentum WiKim0102 (accession number KCCM12356P) or its culture as an active ingredient.

In addition, the present invention provides a lactic acid bacteria starter for fermentation comprising Lactobacillus fermentum WiKim0102 (accession number KCCM12356P) or its culture as an active ingredient.

Furthermore, the present invention provides a method for prevention or treatment of cancer, in which the composition is administered to a subject other than humans.

### [ADVANTAGEOUS EFFECTS]

Lactobacillus fermentum WiKim0102 according to the present invention shows excellent anti-cancer activity in conventional animal models and animal models in which a human cancer cell is transplanted, and therefore it can be usefully used as a composition in use of treatment, prevention or improvement of cancer of humans or animals.

### [BRIEF DESCRIPTION OF THE DRAWINGS]

FIG. 1 is a drawing which shows a photograph of a tumor part of a mouse for observing that the size of tumor cells changes over time after intravenous injection of the composition according to the present invention into CT26 cell transplanted mice once (Single) or 3 times (Serial).
FIG. 2 is a drawing which shows a graph measuring the volume change and growth rate of tumor cells over time after intravenous injection of the composition according to the present invention into CT26 cell transplanted mice once (Single) or 3 times (Serial).
FIG. 3 is a drawing which shows a photograph of a tumor part of a mouse for observing that the size of tumor cells changes over time after intravenous injection of the composition according to the present invention into MC38 cell transplanted mice.
FIG. 4 is a drawing which shows a graph measuring the volume change and growth rate of tumor cells over time after intravenous injection of the composition according to the present invention into MC38 cell transplanted mice.
FIG. 5 is a drawing which shows a graph measuring intra-tissue distribution of Lactobacillus fermentum WiKim0102 in 1 hour and 24 hours after intravenous injection of the composition according to the present invention into CT26 cell transplanted mice.
FIG. 6 is a drawing which shows a graph measuring intra-tissue distribution of Lactobacillus fermentum WiKim0102 in 1 hour and 24 hours after intravenous injection of the composition according to the present invention into MC38 cell transplanted mice.
FIG. 7 is a drawing which shows a photograph of a tumor part of a mouse for observing that the size of tumor cells changes over time after intravenous injection of the composition according to the present invention into HCT116 human colorectal cancer cell transplanted mice once (Single) or 3 times (Serial).
FIG. 8 is a drawing which shows a photograph of a tumor part of a mouse for observing that the size of tumor cells changes over time after intravenous injection of the composition according to the present invention into SW620 human colorectal cancer cell transplanted mice.
FIG. 9 is a drawing which shows a graph measuring the volume change of tumor cells over time after intravenous injection of the composition according to the present invention into HCT116 human colorectal cancer cell transplanted mice.
FIG. 10 is a drawing which shows a graph measuring the volume change of tumor cells over time after intravenous injection of the composition according to the present invention into SW620 human colorectal cancer cell transplanted mice.
FIG. 11 is a drawing which shows a photograph of a tumor part of a mouse for observing that the size of tumor cells changes over time after intravenous injection of the composition according to the present invention into H1650 human non-small cell lung cancer cell transplanted mice.
FIG. 12 is a drawing which shows a graph measuring the volume change of tumor cells over time after intravenous injection of the composition according to the present invention into H1650 human non-small cell lung cancer cell transplanted mice.
FIG. 13 is a drawing which shows a graph measuring the growth rate (cell viability assay) after treating Lactobacillus fermentum WiKim0102 to the human derived pancreatic cancer ASPC1 cell line and PANC1 cell line.
FIG. 14 is a drawing which shows a graph measuring the growth rate (cell viability assay) after treating Lactobacillus fermentum WiKim0102 to the human derived liver cancer HepG2 cell line.
FIG. 15 is a drawing which shows a graph measuring the growth rate (cell viability assay) after treating Lactobacillus fermentum WiKim0102 to the human derived bladder cancer T24 cell line.
FIG. 16 is a drawing which shows a graph measuring the growth rate (cell viability assay) after treating Lactobacillus fermentum WiKim0102 to the mouse derived skin cancer melanoma B16F10 cell line.
FIG. 17 is a drawing which shows a graph measuring the growth rate (cell viability assay) after treating Lactobacillus fermentum WiKim0102 to the human derived normal skin CCD-986-sk cell line.

### [MODE FOR INVENTION]

Hereinafter, the present invention will be described in more detail by examples. These examples are only intended to illustrate the present invention more specifically, and it will be obvious to those skilled in the art that the scope of the present invention is not limited by these samples according to the gist of the present invention.

Through the examples of the present invention, it is shown that the Lactobacillus fermentum strain derived from kimchi has the nucleic acid sequence of SEQ ID NO: 1, as a result of 16S rRNA sequencing for identification and classification of microorganisms.

Accordingly, the microorganism of the present invention which has the 16S rRNA sequence of SEQ ID NO: 1 is named Lactobacillus fermentum WiKim0102, and deposited to Korean Culture Center of Microorganisms on October 31, 2018 (Accession number KCCM12356P).

Herein, 'probiotics' are understood to mean living microorganisms that have a beneficial effect on health of the host by improving the host's intestinal microbial environment in the gastrointestinal tract of animals including humans. Probiotics are living microorganisms with probiotic activity and are in the form of single or complex strains, and when fed to humans or animals in the form of dried cells or fermented products to humans or animals, they can have a beneficial effect on the intestinal flora of the host.

The composition comprising Lactobacillus fermentum WiKim0102 (Accession number KCCM12356P) or its culture as an active ingredient of the present invention has an effect of prevention or treatment of cancer, and may be used as a pharmaceutical composition.

The cancer may be any one selected from the group consisting of bladder cancer, breast cancer, melanoma, thyroid cancer, parathyroid cancer, rectal cancer, throat cancer, laryngeal cancer, esophageal cancer, pancreatic cancer, stomach cancer, tongue cancer, skin cancer, brain tumor, uterine cancer, gallbladder cancer, oral cancer, colon cancer, anal region cancer, liver cancer, lung cancer and colorectal cancer, and preferably, it may be colorectal cancer, lung cancer, pancreatic cancer, liver cancer, bladder cancer or skin cancer, but not limited thereto. The lung cancer may be non-small cell lung cancer.

The Lactobacillus fermentum WiKim0102 comprised in the composition according to the present invention may be present as a live cell or dead cell, and in addition, it may be present in a dried or freeze-dried form. Forms and formulation methods of lactic acid bacteria suitable for inclusion in various compositions are well known to those skilled in the art.

The composition may be administered orally or parenterally. In case of parenteral administration, it may be administered by intravenous injection, subcutaneous injection, intramuscular injection, intraperitoneal injection, endothelial administration, local administration, intranasal administration and rectal administration, and the like, and preferably, it may be administered by intravenous injection, but not limited thereto.

The appropriate dose of the present invention may be prescribed in various ways depending on factors such as formulation method, patient's age, body weight, gender, pathological condition, food, administration time, administration route, excretion rate and response sensitivity.

When the composition of the present invention is utilized as a pharmaceutical composition, the pharmaceutical composition of the present invention may be prepared by using a pharmaceutically appropriate and physiologically acceptable adjuvant in addition to the active ingredient, and as the adjuvant, an excipient, disintegrating agent, sweetener, binding agent, coating material, expansion agent, lubricant, glidant or flavoring agent, or the like may be used.

The pharmaceutical composition may be preferably formulated as a pharmaceutical composition by additionally comprising one or more kinds of pharmaceutically acceptable carriers in addition to the described active ingredient for administration.

For example, for formulation in a form of a tablet or capsule, the active ingredient may be bound to an oral and non-toxic, pharmaceutically acceptable inactive carrier, such as ethanol, glycerol, water and the like. In addition, when desired or needed, a suitable binding agent, a lubricant, a disintegrating agent and a coloring agent may also be comprised in the mixture. The suitable binding agent is not limited thereto, but includes starch, gelatin, natural sugars such as glucose or beta-lactose, natural and synthetic gum such as corn sweetener, acacia, tragacanth or sodium oleate, sodium stearate, magnesium stearate, sodium benzoate, sodium acetate, sodium chloride, and the like. The disintegrating agent is not limited thereto, but includes starch, methyl cellulose, agar, bentonite, xanthan gum, and the like. As the pharmaceutically acceptable carrier in the composition to be formulated as a liquid solution, saline solution, sterile water, Ringer's solution, buffered saline solution, albumin injection solution, dextrose solution, maltodextrin solution, glycerol, ethanol and a mixture of one or more components among them may be used, and if necessary, other common additive such as an anti-oxidant, buffer solution, a bacteriostatic agent, and the like may be added. In addition, it may be formulated as a formulation for injection such as aqueous solution, suspension, emulsion, etc., pill, capsule, granule or tablet, by additionally adding a diluent, a dispersing agent, a surfactant, a binding agent and a lubricant.

Furthermore, as an appropriate method in the field, using the method disclosed in Remington's Pharmaceutical Science, Mack Publishing Company, Easton PA, it may be preferably formulated depending on each disease or component.

The composition comprising Lactobacillus fermentum WiKim0102 (Accession number KCCM12356P) or its culture as an active ingredient of the present invention may be used as a food composition or food additive composition for prevention or improvement of cancer.

The food composition may be a form of health functional food.

The "health functional food" means a food produced and processed using raw materials or ingredient having useful functions to human bodies in accordance with the Health Functional Food Act (Article 3, No. 1), and the "functionality" means to obtain useful effects for health uses such as regulating nutrients or physiological effects, and the like, on the structure and function of human bodies (same Article, No. 2).

The food composition may comprise a food additive additionally, and unless otherwise specified, the suitability as a "food additive" shall be determined according to the standards and criteria for the corresponding item in accordance with the General Rules and General Test Methods of the Food Additive Code approved by the Ministry of Food and Drug Safety.

The item listed in the "Food Additive Code" may include for example, chemical synthetic products such as ketones, glycine, potassium citrate, nicotinic acid, cinnamic acid, etc., natural additives such as Persimmon color, licorice extract, crystalline cellulose, guar gum, etc., and mixed formulations such as L-glutamine sodium formulations, alkali agents for noodles, preservative formulations, tar color formulations, etc.

The food comprising the active ingredient of the present invention may include confectionery such as bread, rice cakes, dried cakes, candies, chocolates, chewing gum and jam, ice cream produces such as ice cream, ice and ice cream powder, dairy products such as milk, low-fat milk, lactose degradation milk, processed milk, goat milk, fermented milk, butter milk, concentrated milk, milk cream, natural cheese, processed cheese, powdered milk and whey, meat products such as processed meat products, processed egg products and hamburgers, fish meat products such as fish cakes, ham, sausage, bacon, etc., noodles such as ramen, dried noodles, fresh noodles, instant fried noodles, gelatinized dried noodles, improved cooked noodles, frozen noodles and pastas, beverages such as fruit juice beverages, vegetable beverages, soybean milk, lactobacillus beverages such as yogurt, etc., and mixed beverages, sauces such as soy sauce, soybean paste, red pepper paste, black soybean paste, mixed paste and vinegar, seasoning food such as tomato ketchup, curry and dressing, margarine, shortening and pizza, but not limited thereto.

In addition thereto, the composition of the present invention may comprise various nutrients, vitamins, electrolytes, flavoring agents, coloring agents, pectic acid and its salts, alginic acid and its salts, organic acids, protective colloid thickeners, pH adjusting agents, stabilizers, preservatives, glycerin, alcohols, carbonating agents used for carbonated beverages, and the like. Moreover, the composition of the present invention may comprise flesh for production of natural fruit juices, fruit juice beverages and vegetable beverages. These ingredients may be used independently or in combination.

The beverage composition comprising the active ingredient of the present invention has no special limitations for other components, and as common beverages, may contain an additional component such as various flavoring agents or natural carbohydrates, or the like. The example of the natural carbohydrates described above common sugars such as monosaccharides (for example, glucose, fructose, etc.); disaccharides (for example, maltose, sucrose, etc.); and polysaccharides (for example, dextrin, cyclodextrin, etc.), and sugar alcohols such as xylitol, sorbitol, erythritol, and the like. As a flavoring agent other than those described above, natural flavoring agents (thaumatin, stevia extracts (for example, rebaudioside A, glycyrrhizin, etc.)) and synthetic flavoring agents (saccharin, aspartame, etc.) are may be advantageously used.

In addition, the composition comprising Lactobacillus fermentum WiKim0102 (Accession No. KCCM12356P) or its culture as an active ingredient of the present invention may be used as a feed composition or feed additive composition for prevention or improvement of cancer of livestock.

When the composition is produced as a feed additive, the composition may be produced as high concentrate of 20 to 90 % or in a powder or granule form. The feed additive may additionally comprise any one, or one or more of organic acids such as citric acid, fumaric acid, adipic acid, lactic acid, malic acid, etc., or phosphates such as sodium phosphate, potassium phosphate, acidic pyrophosphate, polyphosphate (polyphosphate), etc., or natural anti-oxidants such as polyphenol, catechin, alpha-tocopherol, rosemary extract, vitamin C, green tea extract, licorice extract, keto acid, tannic acid, phytic acid, etc. When produced as feed, the composition may be formulated in a common feed form, and may comprise common feed components together.

The feed and feed additive may further comprise grain, for example, powdered or crushed wheat, oat, barley, corn and rice; plant protein feed, for example, feed having rape, bean and sunflower as a main component; animal protein feed, for example, powdered blood, meat meal, bone dust and fish meal; sugars and dairy products, for example, dried components consisting of various kinds of powdered milk and milk serum powder, and the like, and in addition thereto, may further comprise a nutritional supplement, a digestion and absorption enhancer, a growth promoting agent, and the like.

The feed additive may be administered alone or administered in combination with other feed additive in an edible carrier to animals. In addition, the feed additive may be mixed as top-dressing or directly to animal feed, or easily administered to animals as a separate oral formulation from feed. When the feed additive is administered separately from animal feed, as well-known in the art, it may be produced as an immediate release or sustained release formulation, in combination to a pharmaceutically acceptable edible carrier. This edible carrier may be solid or liquid, for example, corn starch, lactose, sucrose, bean flake, peanut oil, olive oil, sesame oil and propylene glycol. When a solid carrier is used, the feed additive may be a tablet, capsule, powder, troche, lozenge, or non-dispersed form of top-dressing. When a liquid carrier is used, the feed additive may be a formulation of gelatin soft capsule, or syrup or suspension, emulsion or solution.

Furthermore, the feed and feed additive may contain a supplement, for example, a preservative, stabilizer, wetting agent or emulsifier, solution promoter, or the like. The feed additive may be used by adding it to animal feed by salivating, spraying or mixing.

The feed or feed additive of the present invention may be applied to many animal feeds including mammals, poultry and fish.

It may be used for pigs, cows, sheep, goats, experimental rodents and pets (e.g.: dogs, cats) in addition to experimental rodents, and the like, as the mammals, and may be used for chickens, turkeys, ducks, geese, pheasants, and quails, and the like, as the poultry, and may be used for trout, and the like as the fish, but not limited thereto.

In addition, the composition comprising Lactobacillus fermentum WiKim0102 (Accession number KCCM12356P) or its culture as an active ingredient of the present invention may be used as a lactic acid bacteria starter for fermentation.

### Example 1. Isolation and culture of strain

### 1-1. Isolation and identification of strain

The present inventors have isolated lactic acid bacteria from kimchi.

At first, a kimchi sample was crushed and then the undiluted solution of kimchi extracts was spread on an MRS agar medium and then cultured at 30°C over 24 hours. A single colony formed after culturing was subcultured to select bacterial single colony, and the selected bacterial single colony was finally identified by 16S rRNA sequencing. In that, one kind of lactic acid bacterium with excellent effects was named Lactobacillus fermentum WiKim0102, given a unique number of WiKim0102 from Microorganism and Gene Bank of World Institute of Kimchi, and was deposited to Korean Culture Center of Microorganisms on October 31, 2018 (Accession No. KCCM12356P).

As the result of 16S rRNA sequencing for identification of microorganisms, it was shown that the strain isolated by the example of the present invention has the nucleic acid sequence of SEQ ID NO: 1.

### 1-2. Culture of strain

The single colony of the Lactobacillus fermentum WiKim0102 strain isolated and identified in the Example 1-1 was under shaking culture at 37°C for 24 hours at 200rpm after inoculating in an MRS liquid medium of 10mℓ. After culturing, the strain cells were centrifuged at 8,000rpm for 5 minutes to remove the culture solution and washed with PBS (Phosphate Buffered Saline) three times to remove the remaining medium components.

### Example 2. Conditions of experimental animal (mouse)

As an experimental animal used for the experiment, male 5-week-old BALB/c mice (Orient Bio, Korea) were supplied, and raised during the experiment period after a stabilization period of 1 week in an animal breeding room in an SPF environment where the room temperature of 20±2°C and humidity of 55±15% were maintained. As a sample, a general pellet sample with no antibiotic was supplied and water was made available for ingestion frequently. For the experimental progression, all animals were bred, experimented and euthanized according to the protocol approved by the Animal Experimental Ethics Committee of World Kimchi Research. The observation of the change in tumor size was progressed by measuring the volume (mm³) of the tumor using an equation of 3.14 x (length x height x area)/6.

### Example 3. Analysis of anti-tumor effect in CT26 and MC38 mouse colorectal cancer cell transplanted animal model

### 3-1. Cell culture

CT26 mouse colorectal cancer cells (Korean Cell Line Bank, Korea) were purchased and used, and MC38 mouse colorectal cancer cells (Chonnam National University, Medical college) were provided and used. The CT26 and MC38 mouse colorectal cancer cells were cultured under the conditions of 5% CO₂ and 37°C in a DMEM medium (Hyclone, U.S.) containing 10% fetal bovine serum and 1% penicillin-streptomycin.

### 3-2. Production of cell transplanted cancer animal model

For production of an animal model of CT26 cell transplantation, 6-week-old BALB/c mice (18-21g) were used for experiment, and for production of an animal model of MC38 cell transplantation, 6-week-old C57BL/6 mice (18-21g) were used. The cultured mouse colorectal cancer cells CT26 and MC38 were injected subcutaneously in the right thigh of the mice, after harvesting 1x10⁵ cell, respectively, and resuspending in PBS of 50µℓ

### 3-3. Analysis of anti-tumor effect

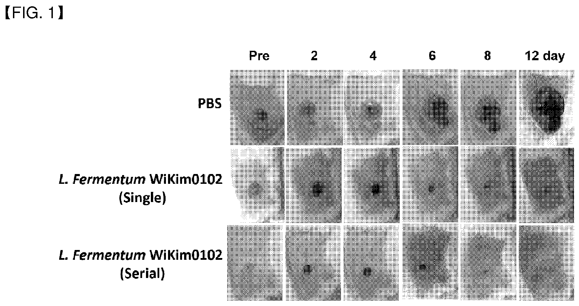

The Lactobacillus fermentum WiKim0102 was intravenously injected to the tail of the CT26 cell transplanted mice in which tumor was formed in a volume of about 80∼100mm³. The Lactobacillus fermentum WiKim0102 was prepared by quantifying the number of bacteria in 1×10¹⁰ CFU/mℓ using PBS, and 0.1mℓ (1×10⁹ CFU) was injected intravenously into the tail once or three times to the experimental animal, and PBS was administered to the negative control group. The result of confirming the change in the colorectal cancer tumor size of the CT26 cell transplanted mice with naked eyes over time was shown in FIG. 1.

As shown in FIG. 1, it was observed that the tumor cells were reduced to a degree that could be seen with naked eyes, after 12 days, when the Lactobacillus fermentum WiKim0102 was intravenously injected, compared to the negative control group (PBS). In particular, it was confirmed that the tumor size was much more reduced when it was injected three times (Serial) than once (Single), and it was determined that the tumor was reduced to a degree that could not be seen with naked eyes when 12 days were over when it was injected three times.

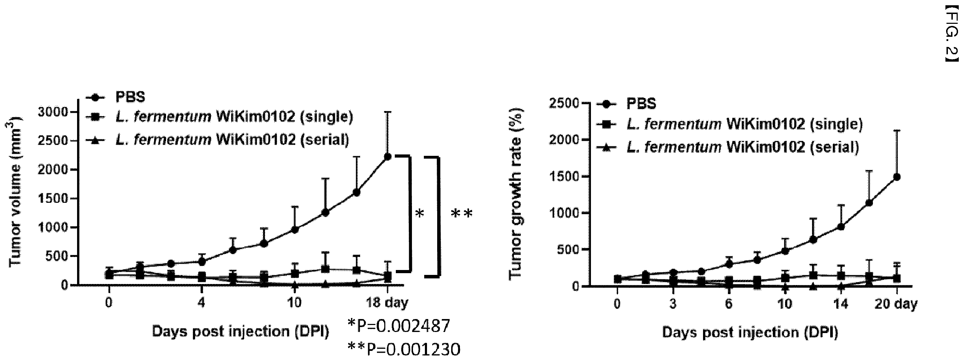

In addition, the result of measuring the volume change in tumor size in the CT26 cell transplanted mice over time was shown in FIG. 2.

As shown in FIG. 2, it could be confirmed that in the colorectal cancer tumor size of the CT26 cell transplanted mice, there were about 4.2 times of anti-tumor effects, in 18 days, when the Lactobacillus fermentum WiKim0102 was injected once, compared to the negative control group, and when it was injected three times at an interval of 6 days, in 18 days, about 27.2 times of anti-tumor effects were observed, compared to the negative control group.

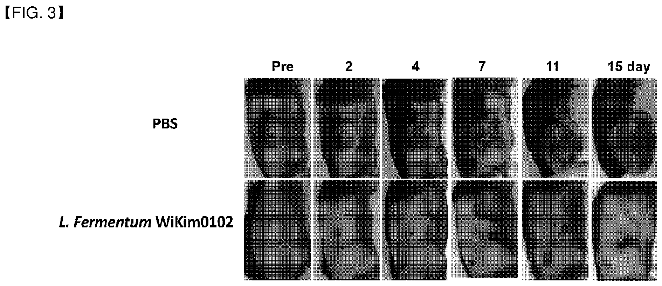

Furthermore, the Lactobacillus fermentum WiKim0102 was intravenously injected to the tail of the MC38 cell transplanted mice in which tumor was formed in a volume of about 80∼100mm³. The Lactobacillus fermentum WiKim0102 was prepared by quantifying the number of bacteria in 1×10¹⁰ CFU/mℓ using PBS, and 0.1 mℓ (1×10⁹ CFU) was injected intravenously into the tail once or three times to the experimental animal, and PBS was administered to the negative control group. The result of confirming the change in the colorectal cancer tumor size of the Mc38 cell transplanted mice with naked eyes over time was shown in FIG. 3.

As shown in FIG. 3, it was observed that the tumor cells were reduced to a degree that could be seen with naked eyes, when the Lactobacillus fermentum WiKim0102 was intravenously injected, compared to the negative control group (PBS). It could be observed that the tumor was reduced to a degree that could not be seen with naked eyes when 15 days were over.

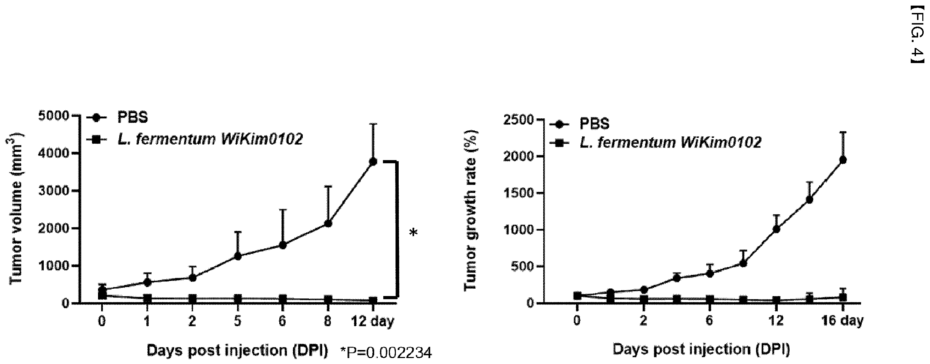

In addition, the result of measuring the volume change in tumor size in the MC38 cell transplanted mice over time was shown in FIG. 4.

As shown in FIG. 4, it could be confirmed that in the colorectal cancer tumor size of the MC68 cell transplanted mice, there were about 23.8 times of anti-tumor effects, in 12 days, when the Lactobacillus fermentum WiKim0102 was injected once, compared to the negative control group.

Through the experimental results, the present inventors could determine that the anti-tumor effect of the Lactobacillus fermentum WiKim0102 was excellent, and in particular, in case of continuous administration, it showed the more excellent anti-tumor effect.

### Example 4. Biodistribution study after injection of Lactobacillus fermentum WiKim0102

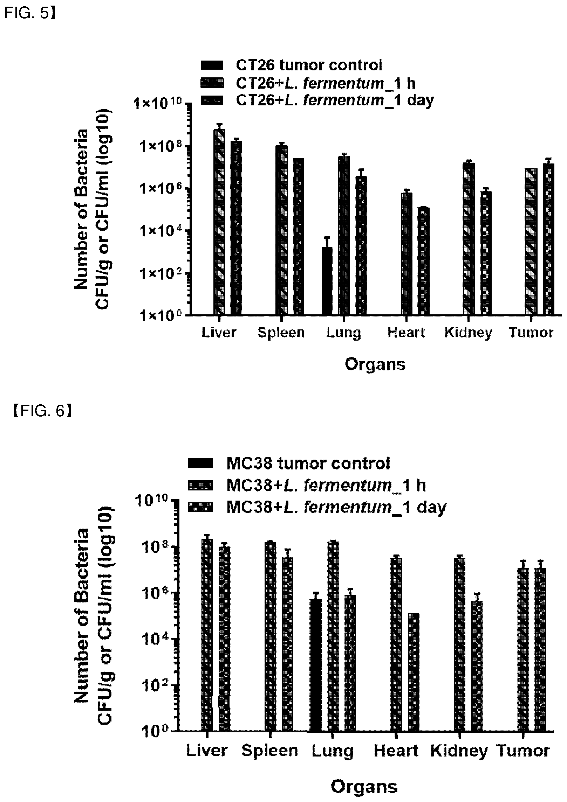

In 1 hour and 24 hours after injecting the Lactobacillus fermentum WiKim0102 intravenously to the tail of CT26 cell and MC38 cell transplanted mice, the tumor tissue was removed and homogenized, and then spread in an MRS agar medium. The colony formed by culturing at 37°C for 24 hours after spreading was counted to calculate CFU/g tissue. In order to compare the degree of targeting Lactobacillus fermentum WiKim0102 of the tumor tissue and other major organ tissue, the hepatic and spleen tissues were also homogenized under the same condition to count bacteria, and the result was shown in FIG. 5 (CT26 cell transplanted mice) and FIG. 6 (MC38 cell transplanted mice).

As shown in FIG. 5 and FIG. 6, it was confirmed that in all cases of cancer animal models in which CT26 and MC38 cells were transplanted, from 1 hour after the Lactobacillus fermentum WiKim0102 was injected, the bacterial count of 1×10⁷ CFU/g or more was observed in the tumor tissue, and in 24 hours, the bacterial count of the tumor tissue was maintained.

From the result, it could be determined that the Lactobacillus fermentum WiKim0102 was accumulated in the tumor tissue and effectively targeted, in the mouse models in which the CT26 cell and MC38 cell were transplanted.

### Example 5. Analysis of anti-tumor effect in HCT116, SW620 (human colorectal cancer) cell and H1650 (human non-small cell lung cancer) cell transplanted animal model

### 5-1. Cell culture

HCT116 human colorectal cancer cells are SW620 human colorectal cancer cells (Korean Cell Line Bank, Korea) were purchased and used, and H1650 human non-small cell lung cancer cells (Chonnam National University, Medical college) were provided and used. These HCT116, SW620 and H1650 cells were cultured under the conditions of 5% CO₂ and 37°C in an RPMI 1640 medium (Hyclone, U.S.) containing 10% fetal bovine serum and 1% penicillin-streptomycin.

### 5-2. Production of HCT116, SW620 and H1650 cells transplanted cancer animal models

For production of animal models of HCT116, SW620 and H1650 cells transplantation, 6-week-old BALB/c mice (17-20g) were used for experiment. The cultured HCT116, SW620 and H1650 cells were injected subcutaneously in the right thigh of the mice, after harvesting 5x10⁶ cell, respectively, and resuspending in PBS of 50µℓ.

### 5-3. Analysis of anti-tumor effect for HCT116, SW620 colorectal cancer cells

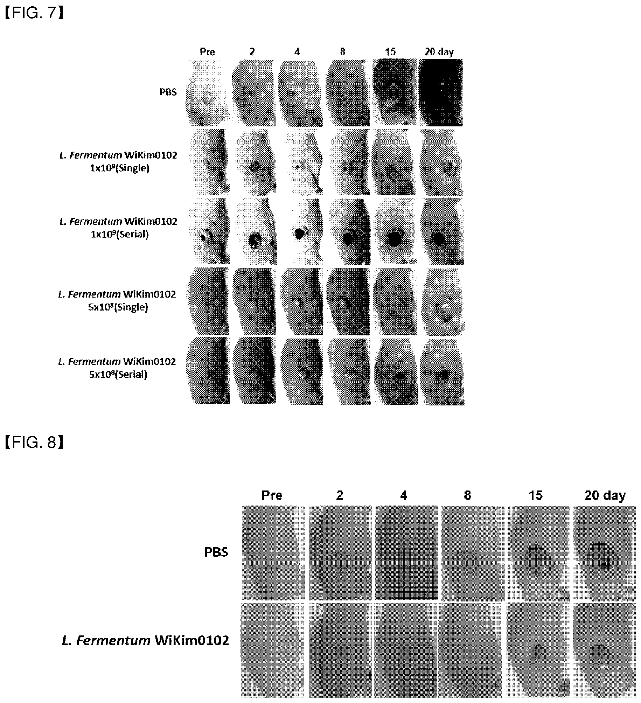

The Lactobacillus fermentum WiKim0102 was intravenously injected to the tail of the HCT116 and SW620 colorectal cancer cells transplanted mice in which tumor was formed in a volume of about 80∼100mm³. The Lactobacillus fermentum WiKim0102 was prepared by quantifying the number of bacteria in 1×10¹⁰ CFU/mℓ or 5×10⁹ CFU/mℓ using PBS. In the cancer animal model in which the HCT116 human colorectal cancer cell was transplanted, the Lactobacillus fermentum WiKim0102 was injected intravenously into the tail in an amount of 0.1 mℓ (1×10⁹ CFU) once or three times at an interval of 6 days or in an amount of 0.1 mℓ (5×10⁸ CFU) once or three times at an interval of 6 days. PBS was administered to the negative control group in the same way, and as the result of confirming the change in the tumor size over time for 26 days with naked eyes and the volume change in the tumor size were shown in FIG. 7 to FIG. 10.

As shown in FIG. 7 and FIG. 8, it was observed that the tumor size was reduced to a degree that it could be observed with naked eyes, when the Lactobacillus fermentum WiKim0102 was injected to the cancer animal models in which HCT116 and SW620 human colorectal cancer cells were transplanted, and it was confirmed that the tumor reduction effect was excellent when the Lactobacillus fermentum WiKim0102 was administered in an amount of 0.1mℓ(1×10⁹ CFU), than the case that it was administered in an amount of 0.1mℓ (5×10⁸CFU). In particular, it was determined that the reduction effect of the tumor size was excellent in case of three times of administration than single administration in the cancer animal model in which the HCT116 human colorectal cancer cell was transplanted.

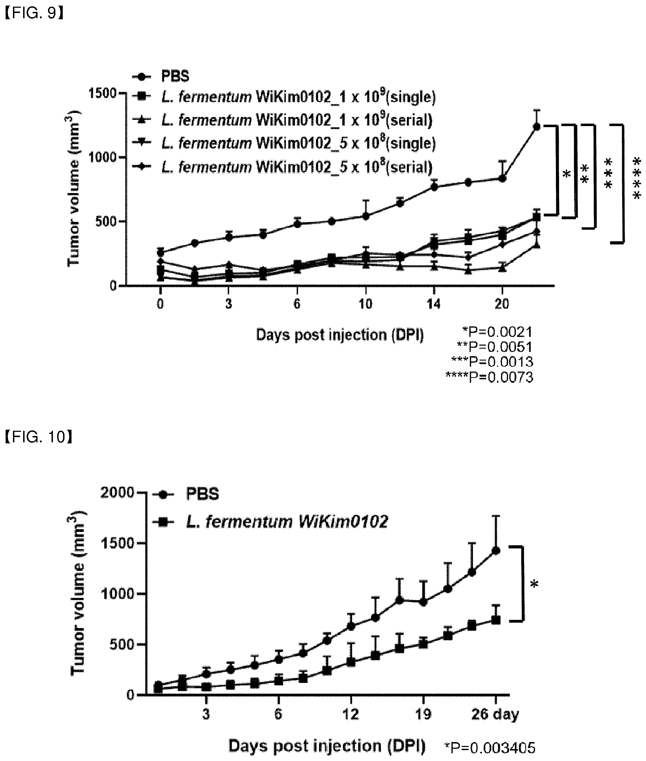

In addition, as shown in FIG. 9 and FIG. 10, it could be numerically confirmed that the volume in tumor size was definitely reduced when the Lactobacillus fermentum WiKim0102 was injected to the cancer animal models in which the HCT116 and SW620 human colorectal cancer cells were transplanted. When the Lactobacillus fermentum WiKim0102 was injected into the cancer animal model in which the HCT116 human colorectal cancer cell was transplanted in an amount of 1×10⁹ CFU/0.1 mℓ once or three times, in 26 days, about 4.2 times and about 27.2 times of anti-tumor effects were observed, respectively, compared to the negative control group, and when the Lactobacillus fermentum WiKim0102 was injected in an amount of 5×10⁸CFU/0.1mℓ three times, in 26 days, about 2.9 times of anti-tumor effects were observed, compared to the negative control group. On the other hand, when the Lactobacillus fermentum WiKim0102 was injected into the cancer animal model in which the SW620 human colorectal cancer cell was transplanted was injected in an amount of 1×10⁹ CFU/0.1mℓ once, in 26 days, about 2.1 times of anti-tumor effects were observed, compared to the negative control group.

From the result, when the Lactobacillus fermentum WiKim0102 was injected three times, than once, a more excellent effect was shown in inhibiting the tumor growth. Accordingly, it was confirmed that injection administration according to appropriate intervals is more effective than one injection for effective treatment of tumor, and it has excellent anti-cancer activity not only in mouse colorectal cancer cells but also in human colorectal cancer cells transplanted animal models.

### 5-4. Analysis of anti-tumor effect for H1650 non-small cell lung cancer

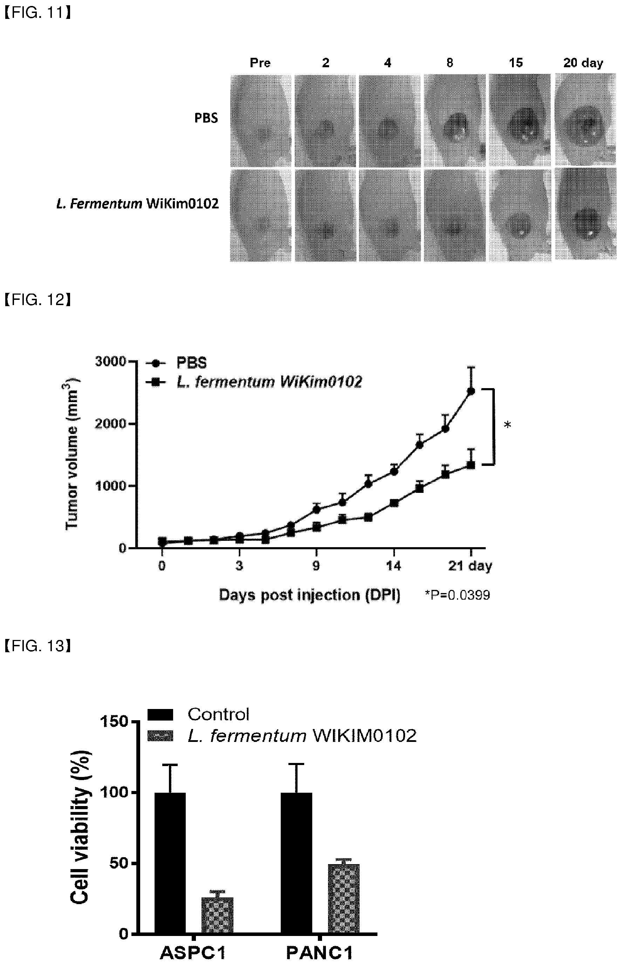

The Lactobacillus fermentum WiKim0102 was intravenously injected to the tail of the H1650 non-small cell lung cancer cell transplanted mice in which tumor was formed in a volume of about 80∼100mm³. The Lactobacillus fermentum WiKim0102 was prepared by quantifying the number of bacteria in 1×10¹⁰ CFU/mℓ or 5×10⁹ CFU/mℓ using PBS. In the cancer animal model in which the H1650 non-small cell lung cancer cell was transplanted, the Lactobacillus fermentum WiKim0102 was injected intravenously into the tail in an amount of 0.1mℓ (1×10⁹ CFU) once. PBS was administered to the negative control group in the same way, and as the result of confirming the change in the tumor size over time for 21 days with naked eyes and the graph showing the volume change in the tumor size were shown in FIG. 11 to FIG. 12.

As shown in FIG. 11, it was observed that the tumor size was reduced to a degree that it could be observed with naked eyes, when the Lactobacillus fermentum WiKim0102 was injected to the cancer animal model in which the H1650 non-small cell lung cancer cell was transplanted.

In addition, as shown in FIG. 12, it could be numerically confirmed that the volume in tumor size was definitely reduced when the Lactobacillus fermentum WiKim0102 was injected to the cancer animal model in which the H1650 non-small cell lung cancer cell was transplanted. When the Lactobacillus fermentum WiKim0102 was injected to the cancer animal model in which the H1650 non-small cell lung cancer cell was transplanted, in 21 days, about 1.8 times of anti-tumor effects were observed, compared to the negative control group.

### Example 6. In vitro anti-cancer activity efficacy analysis

### 6-1. Preparation of cell lines

In order to observe the anti-cancer activity effect of the Lactobacillus fermentum WiKim0102, the cell growth rate (cell viability assay) was measured using Cell counting Kit-8. As cells used for the anti-cancer activity efficacy experiment, human-derived pancreatic cancer ASPC1 and PANC1 cell lines, human-derived liver cancer HepG2 cell line, human-derived bladder cancer T24 cell line, mouse-derived skin melanoma B16F10 cell line and normal skin cell line, CCD-986-sk cell line were used.

The human-derived cancer cells, ASPC1, PANC1, HepG2 and T24 cell lines were subcultured under the conditions of 5% CO₂, and 37°C using an RPMI medium in which 10% FBS (fetal bovine serum) and 1% penicillin/streptomycin were added, and the cells in the growth period were used for the experiment. The cultured ASPC1, PANC1, HepG2 and T24 cells were treated with 0.25% trypsin-EDTA in an incubator at 37°C for 3 minutes to detach the cells and then they were washed with DPBS twice and prepared as 1 X 10⁴ cells/well.

The mouse-derived skin melanoma B16F10 cell line was subcultured under the conditions of 5% CO₂, and 37°C using a DMEM medium in which 10% FBS (fetal bovine serum) and 1% penicillin/streptomycin were added and the cells in the growth period were used for the experiment. The cultured B16F10 cells were treated with 0.25% trypsin-EDTA in an incubator at 37°C for 3 minutes to detach the cells and then they were washed with DPBS twice and prepared as 1 X 10⁴ cells/well.

The human-derived normal skin cell CCD-986-sk cell line was subcultured under the conditions of 5% CO₂, and 37°C using an RPMI medium in which 10% FBS (fetal bovine serum) and 1% penicillin/streptomycin were added and the cells in the growth period were used for the experiment. The cultured CCD-986-sk cells were treated with 0.25% trypsin-EDTA in an incubator at 37°C for 3 minutes to detach the cells and then they were washed with DPBS twice and prepared as 1 X 10⁴ cells/well.

### 6-2. Cell growth rate (cell viability assay) measurement result

The present invention measured the cell growth rate (cell viability assay) of cells treated with the Lactobacillus fermentum WiKim0102 after culturing pancreatic cancer (2 kinds), liver cancer (1 kind), bladder cancer (1 kind) and skin cancer (1 kind) cells, and confirmed the anti-cancer activity efficacy on the basis of the absorbance of cancer cells not treated with the Lactobacillus fermentum WiKim0102 as the growth rate of 100%.

For the cell growth rate (cell viability assay), they were treated at a concentration of MOI1 and then cultured for 72 hours, and after culturing, they were treated with Cell counting Kit-8 reagent and then the absorbance (OD 450nm) was measured. Based on the absorbance of cells (control) not treated with Lactobacillus fermentum WiKim0102 as 100%, the growth rate of cells treated with Lactobacillus fermentum WiKim0102 was calculated, and the experimental result was described below.

The result of measuring the cell growth rate by treating the Lactobacillus fermentum WiKim0102 to the human-derived pancreatic cancer ASPC1 cell line and human-derived pancreatic cancer cell line PANC1 was shown in FIG. 13.

As shown in FIG. 13, the cell growth rate of 26.3% for the human-derived pancreatic cancer ASPC1 cell line and 49.4% for the human-derived pancreatic cancer cell line PANC1 which were treated with the Lactobacillus fermentum WiKim0102 were measured. From the result, it can be confirmed that it has excellent anti-cancer activity efficacy for pancreatic cancer, as the cell growth rate is significantly inhibited in case of the pancreatic cancer cell lines treated with the Lactobacillus fermentum WiKim0102.

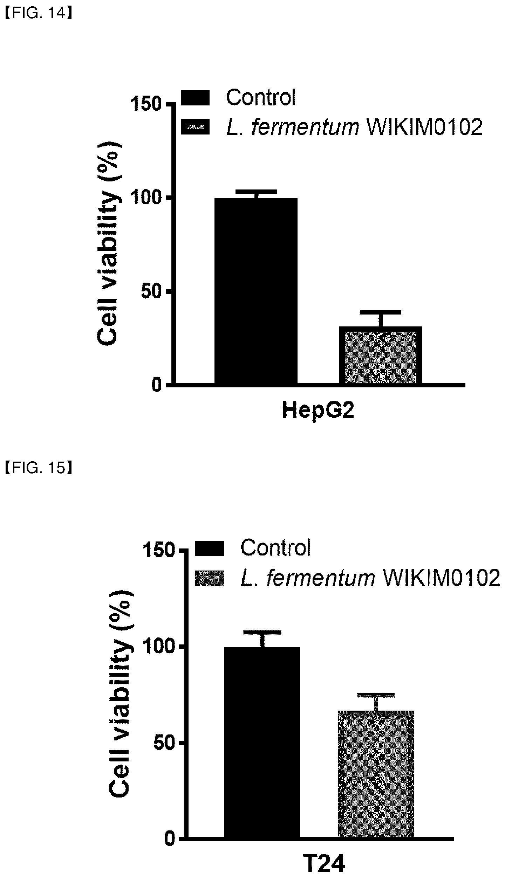

In addition, the result of measuring the cell growth rate by treating the Lactobacillus fermentum WiKim0102 to the human-derived liver cancer HepG2 cell line was shown in FIG. 14.

As shown in FIG. 14, for the human-derived liver cancer HepG2 cell line treated with the Lactobacillus fermentum WiKim0102, the cell growth rate of 31.4% was measured. From the result, it can be confirmed that it has excellent anti-cancer activity efficacy for liver cancer, as the cell growth rate is significantly inhibited in case of the liver cancer cell line treated with the Lactobacillus fermentum WiKim0102.

Furthermore, the result of measuring the cell growth rate by treating the Lactobacillus fermentum WiKim0102 to the human-derived bladder cancer T24 cell line was shown in FIG. 15.

As shown in FIG. 15, for the human-derived bladder cancer T24 cell line treated with the Lactobacillus fermentum WiKim0102, the cell growth rate of 66.8% was measured. From the result, it can be confirmed that it has excellent anti-cancer activity efficacy for bladder cancer, as the cell growth rate is significantly inhibited in case of the bladder cancer cell line treated with the Lactobacillus fermentum WiKim0102.

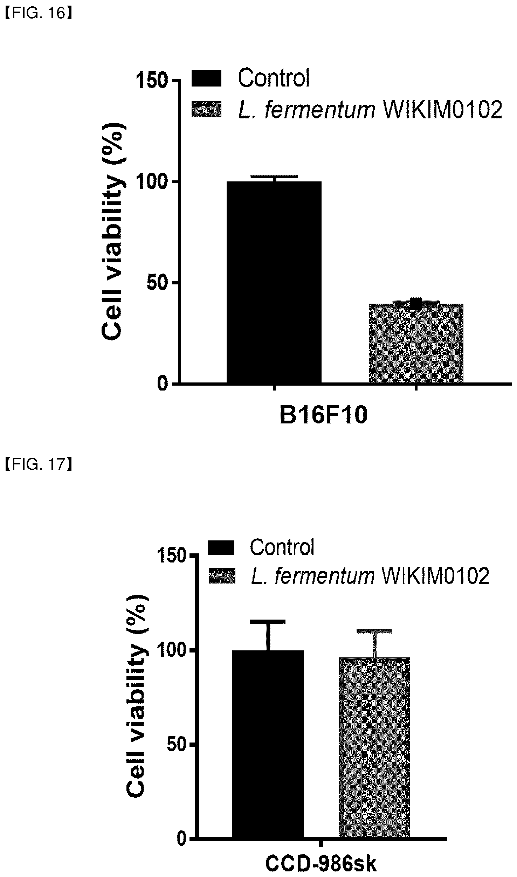

Moreover, the result of measuring the cell growth rate by treating the Lactobacillus fermentum WiKim0102 to the mouse-derived skin melanoma B16F10 cell line was shown in FIG. 16.

As shown in FIG. 16, for the mouse-derived skin melanoma B16F10 cell line treated with the Lactobacillus fermentum WiKim0102, the cell growth rate of 39.7% was measured. From the result, it can be confirmed that it has excellent anti-cancer activity efficacy for skin cancer, as the cell growth rate is significantly inhibited in case of the skin cancer cell line treated with the Lactobacillus fermentum WiKim0102.

Compared to the above experiments, the result of measuring the cell growth rate by treating the Lactobacillus fermentum WiKim0102 to the human-derived normal skin CCD-986-sk cell line was shown in FIG. 17.

As shown in FIG. 17, for the normal skin cell line treated with the Lactobacillus fermentum WiKim0102, the cell growth rate of 96.2% was measured, and from the result, it can be confirmed that the Lactobacillus fermentum WiKim0102 has no toxicity for the normal skin cell line.

The two-tailed t-test determined a statistically significant difference in tumor growth between the negative control group and Lactobacillus fermentum WiKim0102 treatment group. P < 0.05 was significantly considered for all tumor growth graph analyses.

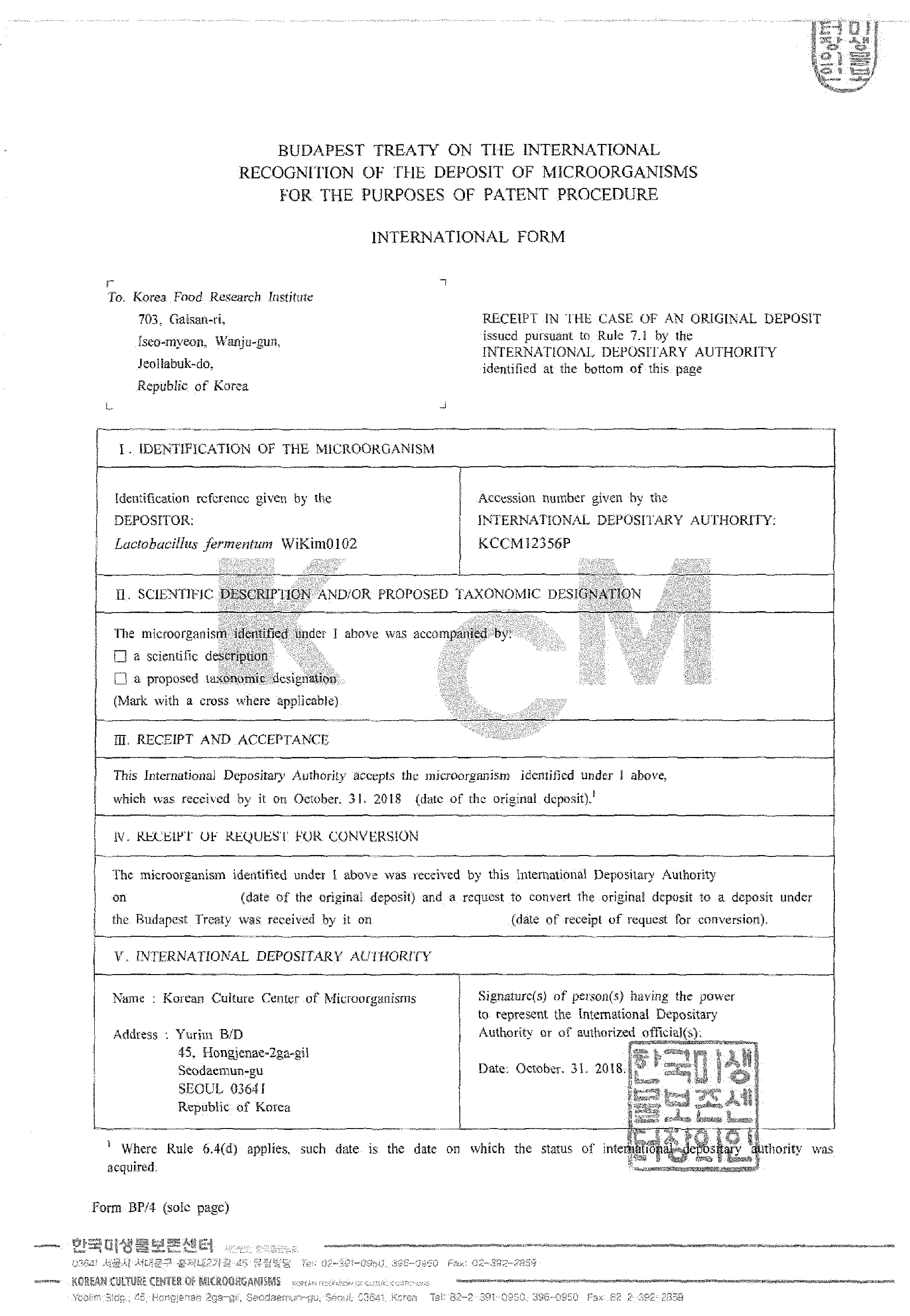
BUDAPEST TREATY ON THE INTERNATIONAL
RECOGNITION OF THE DEPOSIT OF MICROORGANISMS
FOR THE PURPOSES OF PATENT PROCEDURE

INTERNATIONAL FORM

To. Korea Food Research Institute
703, Galsan-ri,
Iseo-myeon, Wanju-gun,
Jeollabuk-do,
Republic of Korea

RECEIPT IN THE CASE OF AN ORIGINAL DEPOSIT
issued pursuant to Rule 7.1 by the
INTERNATIONAL DEPOSITARY AUTHORITY
identified at the bottom of this page

I. IDENTIFICATION OF THE MICROORGANISM
Identification reference given by the
DEPOSITOR:
Lactobacillus fermentum WiKim0102 | Accession number given by the
INTERNATIONAL DEPOSITARY AUTHORITY:
KCCM12356P
II. SCIENTIFIC DESCRIPTION AND/OR PROPOSED TAXONOMIC DESIGNATION
The microorganism identified under I above was accompanied by:
☐ a scientific description
☐ a proposed taxonomic designation
(Mark with a cross where applicable)
III. RECEIPT AND ACCEPTANCE
This International Depositary Authority accepts the microorganism identified under I above,
which was received by it on October. 31. 2018 (date of the original deposit).¹
IV. RECEIPT OF REQUEST FOR CONVERSION
The microorganism identified under I above was received by this International Depositary Authority
on (date of the original deposit) and a request to convert the original deposit to a deposit under
the Budapest Treaty was received by it on (date of receipt of request for conversion).
V. INTERNATIONAL DEPOSITARY AUTHORITY
Name : Korean Culture Center of Microorganisms

Address : Yurim B/D
45, Hongjenae-2ga-gil
Seodaemun-gu
SEOUL 03641
Republic of Korea | Signature(s) of person(s) having the power
to represent the International Depositary
Authority or of authorized official(s):

Date: October. 31. 2018.

¹ Where Rule 6.4(d) applies, such date is the date on which the status of international depositary authority was
acquired.

Form BP/4 (sole page)

한국미생물보존센터 [?]
03641 서울시 서대문구 홍제내2가길 45 유림빌딩 Tel: 02-391-0950, 396-0950 Fax: 02-392-2859
KOREAN CULTURE CENTER OF MICROORGANISMS [?]
Yoolim Bldg., 45, Hongjenae 2ga-gil, Seodaemun-gu, Seoul, 03641, Korea Tel: 82-2-391-0950, 396-0950 Fax: 82-2-392-2859

## Claims

1. Lactobacillus fermentum WiKim0102 of accession number KCCM12356P, having a 16S rRNA represented by SEQ ID NO: 1.

2. A pharmaceutical composition for prevention or treatment of cancer comprising Lactobacillus fermentum WiKim0102 (accession number KCCM12356P) or its culture as an active ingredient.

3. The pharmaceutical composition for prevention or treatment of cancer according to claim 2,
wherein the cancer is any one cancer selected from the group consisting of bladder cancer, breast cancer, melanoma, thyroid cancer, parathyroid cancer, rectal cancer, throat cancer, laryngeal cancer, esophageal cancer, pancreatic cancer, stomach cancer, tongue cancer, skin cancer, brain tumor, uterine cancer, gallbladder cancer, oral cancer, colon cancer, anal region cancer, liver cancer, lung cancer and colorectal cancer.

4. The pharmaceutical composition for prevention or treatment of cancer according to claim 3,
wherein the lung cancer is non-small cell lung cancer.

5. The pharmaceutical composition for prevention or treatment of cancer according to claim 2,
wherein the composition can be administered by a method of oral administration or parenteral administration.

6. The pharmaceutical composition for prevention or treatment of cancer according to claim 5,
wherein the method of parenteral administration is intravenous injection administration.

7. The pharmaceutical composition for prevention or treatment of cancer according to claim 2,
wherein the composition is in a living cell form.

8. A food composition for prevention or improvement of cancer comprising Lactobacillus fermentum WiKim0102 (accession number KCCM12356P) or its culture as an active ingredient.

9. The food composition for prevention or improvement of cancer according to claim 8,
wherein the food is health functional food.

10. The food composition for prevention or improvement of cancer according to claim 8,
wherein the food is any one food selected from the group consisting of bread, rice cake, candy, chocolate, gum, ice cream, milk, cheese, processed meat products, processed fish products, kimchi, soy sauce, soybean paste, red pepper paste, black bean paste, fermented soybean paste, vinegar, ketchup, curry, dressing, beverages and fermented milk.

11. A food additive composition for prevention or improvement of cancer comprising Lactobacillus fermentum WiKim0102 (accession number KCCM12356P) or its culture as an active ingredient.

12. A feed composition for prevention or improvement of cancer of livestock comprising Lactobacillus fermentum WiKim0102 (accession number KCCM12356P) or its culture as an active ingredient.

13. The feed composition for prevention or improvement of cancer of livestock according to claim 12,
wherein the livestock is any one selected from the group consisting of pig, cow, horse, sheep, rabbit, goat, mouse, hamster, guinea pig, dog, cat, chicken, turkey, duck, goose, pheasant, quail, carp, crucian carp and trout.

14. A feed additive composition for prevention or improvement of cancer of livestock comprising Lactobacillus fermentum WiKim0102 (accession number KCCM12356P) or its culture as an active ingredient.

15. A lactic acid bacteria starter for fermentation comprising Lactobacillus fermentum WiKim0102 (accession number KCCM12356P) or its culture as an active ingredient.

16. A method for prevention or treatment of cancer, in which the composition of any one of claims 2 to 7 is administered to a subject other than humans.

17. The method for prevention or treatment of cancer according to claim 16,
wherein the method and dose of administration is to administer 0.1 mℓ of Lactobacillus fermentum WiKim0102 at a concentration of 1×10¹⁰ CFU/mℓ by intravenous injection.

18. The method for prevention or treatment of cancer according to claim 17,
wherein the period and number of administration is administering three times at an interval of 6 days.


<110>    Korea Food Research Institute

<120>    Lactobacillus fermentum WiKim0102 having anti-cancer activity and
         composition for comprising the same

<130>    KP181207-03_WiKim0102

<160>    1

<170>    KoPatentIn 3.0

<210>    1
<211>    1383
<212>    DNA
<213>    Lactobacillus fermentum

<400>    1
cacctgattg attttggtcg ccaacgagtg gcggacgggt gagtaacacg taggtaacct        60

gcccagaagc gggggacaac atttggaaac agatgctaat accgcataac aacgttgttc       120

gcatgaacaa cgcttaaaag atggcttctc gctatcactt ctggatggac ctgcggtgca       180

ttagcttgtt ggtggggtaa ggcctaccaa ggcgatgatg catagccgag ttgagagact       240

gatcggccac aatgggactg agacacggcc catactccta cgggaggcag cagtagggaa       300

tcttccacaa tgggcgcaag cctgatggag caacaccgcg tgagtgaaga agggtttcgg       360

ctcgtaaagc tctgttgtta aagaagaaca cgtatgagag taactgttca tacgttgacg       420

gtatttaacc agaaagtcac ggctaactac gtgccagcag ccgcggtaat acgtaggtgg       480

caagcgttat ccggatttat tgggcgtaaa gagagtgcag gcggttttct aagtctgatg       540

tgaaagcctt cggcttaacc ggagaagtgc atcggaaact ggataacttg agtgcagaag       600

agggtagtgg aactccatgt gtagcggtgg aatgcgtaga tatatggaag aacaccagtg       660

gcgaaggcgg ctacctggtc tgcaactgac gctgagactc gaaagcatgg gtagcgaaca       720

ggattagata ccctggtagt ccatgccgta aacgatgagt gctaggtgtt ggagggtttc       780

cgcccttcag tgccggagct aacgcattaa gcactccgcc tggggagtac gaccgcaagg       840

ttgaaactca aaggaattga cgggggcccg cacaagcggt ggagcatgtg gtttaattcg       900

aagctacgcg aagaacctta ccaggtcttg acatcttgcg ccaaccctag agatagggcg       960

tttccttcgg gaacgcaatg acaggtggtg catggtcgtc gtcagctcgt gtcgtgagat      1020

gttgggttaa gtcccgcaac gagcgcaacc cttgttacta gttgccagca ttaagttggg      1080

cactctagtg agactgccgg tgacaaaccg gaggaaggtg gggacgacgt cagatcatca      1140

tgccccttat gacctgggct acacacgtgc tacaatggac ggtacaacga gtcgcgaact      1200

cgcgagggca agcaaatctc ttaaaaccgt tctcagttcg gactgcaggc tgcaactcgc      1260

ctgcacgaag tcggaatcgc tagtaatcgc ggatcagcat gccgcggtga atacgttccc      1320

gggccttgta cacaccgccc gtcacaccat gagagtttgt aacacccaaa gtcggtgggg      1380

taa                                                    1383